# DEMANDE DE BREVET EUROPEEN

(11) **EP 2 623 051 A2**
(43) Date de publication de la demande: **07.08.2013**
(21) Numéro de dépôt: 13290016.8
(22) Date de dépôt: 21.01.2013
(51) Int. Cl.: A61B 17/16

(54) **Instrument chirurgical d'enlèvement de matière osseuse à dentures pointues**

(30) Priorité: 02.02.2012 FR 1200310
(71) Demandeur: Biegun, Jean-Francois, 90800 Bavilliers (FR)
(72) Inventeur: Biegun, Jean-Francois, 90800 Bavilliers (FR)
(74) Mandataire: Eidelsberg, Olivier Nathan

(57) **Abrégé**

Instrument chirurgical d'enlèvement de matière osseuse ayant une surface de laquelle est issue au moins une denture, de préférence des dentures en matière plastique destinées à venir en contact avec l'os pour en enlever une partie, caractérisé en ce que la largeur (1) de la ou de chaque denture (5), c'est-à-dire la dimension en largeur la plus petite au niveau de ladite une surface de l'instrument dont elle est issue, est comprise entre 1 mm et 7 mm, de préférence comprise entre 2 mm et 5 mm, plus préférablement comprise entre 3 mm et 4 mm.

## Description

La présente invention se rapporte à un procédé de fabrication d'un instrument chirurgical, notamment du type qui présente une zone destinée à attaquer un os pour en enlever une partie, par exemple en le coupant ou en le râpant, par exemple une râpe fémorale, une fraise à cotyle, une râpe de hanche ou analogue. La présente invention se rapporte également à un procédé de fabrication d'un dispositif chirurgical destiné à supporter une contrainte élevée lors de son utilisation, soit lorsqu'il attaque de l'os comme ci-dessus, soit lorsqu'il supporte un instrument chirurgical qui réalise l'attaque de l'os, par exemple un dispositif de guide de coupe. La présente invention se rapporte aussi à un instrument chirurgical et à un dispositif chirurgical, qui peuvent notamment être obtenus par un procédé de fabrication de ce genre.

On connaît déjà de l'art antérieur, et notamment de la demande internationale WO 2004/047655 au nom de la demanderesse, des instruments ou dispositifs chirurgicaux de ce genre dont des parties qui sont destinées à subir de fortes contraintes lorsqu'ils attaquent l'os pour en enlever une partie sont en matière plastique. Ces parties peuvent notamment comporter des dentures réalisées en matière plastique et peuvent être fabriquées par moulage.

Cela permet de les fabriquer en grande quantité à faible coût et ainsi favorise une utilisation à usage unique qui empêche une re-stérilisation, a priori interdite.

On connaît de US-A-5,665,091 une râpe de hanche dont les dentures peuvent être réalisées en matière polymère. Cependant, la fabrication de ces râpes se fait uniquement par usinage. Dans le cas, non décrit dans ce document de l'art antérieur, où l'on réaliserait cette râpe par moulage, on n'obtiendrait pas des dentures suffisamment acérées, seul l'usinage (de préférence d'ailleurs d'une matière métallique) permettant d'obtenir les dentures acérées décrites dans ce document de l'art antérieur. En effet, bien que ces instruments et dispositifs chirurgicaux de l'art antérieur présentent un avantage indéniable par rapport à ce qui existait précédemment, notamment en ce que, bien que réalisés en matière plastique, ils permettent de râper ou couper de l'os et/ou supporter les contraintes liées à de telles actions, on aimerait bien améliorer encore plus leur aptitude à couper et/ou à supporter des contraintes et ce tout en les fabriquant par un procédé de moulage simple et approprié à une production de masse.

La présente invention vise ainsi un procédé de fabrication d'un instrument ou d'un dispositif chirurgical pour attaquer de l'os pour en enlever une partie, dont une partie destinée à supporter des contraintes élevées liées à cette attaque est en matière plastique, cette dite une partie plastique pouvant notamment comporter au moins une denture, de préférence une pluralité de dentures faisant saillie à l'extérieur de l'instrument, et destinée(s) à attaquer l'os directement, tel que défini à l'une des revendications 1 à 7.

De préférence, le volume de la première partie est plus grand que le volume de la partie plastique destinée à supporter les contraintes élevées, notamment est nettement plus grande.

En prévoyant d'effectuer le moulage de la partie plastique supportant les contraintes élevées sur la base d'un moulage d'un petit volume par rapport au volume total de l'instrument, on s'assure d'une meilleure capacité de la partie plastique à supporter les contraintes élevées, notamment sa capacité à attaquer l'os. Ainsi, par exemple dans le cas d'une partie plastique qui comporte des dentures de coupe ou de râpe, les inventeurs ont compris pour la première fois que ce qui limitait la capacité à râper des râpes de l'art antérieur consistait en le fait qu'on ne pouvait pas former par moulage des dentures ayant une petite largeur et/ou une grande hauteur et donc un profil acéré, car la matière plastique coulée dans le moule ne parvenait pas à pénétrer jusqu'au fond des cavités de moulage prévues pour former les dentures. Ainsi, dans l'art antérieur les dentures, une fois extirpées de leur moule, n'avaient pas le profil approprié pour être aussi efficaces que souhaité. Suivant l'invention, pour la première fois on a obtenu en particulier un instrument chirurgical obtenu par moulage avec des dentures en matières plastiques qui conviennent particulièrement bien pour attaquer de l'os.

Suivant un mode de réalisation de l'invention, on place la préforme dans le moule pour former l'instrument final et on fait couler la matière plastique dans le volume restant, incluant, s'il y en a, le volume des dentures, les dimensions du moule par rapport à celles de la préforme étant choisies pour que ce volume restant soit petit, le produit final obtenu comportant alors un interface entre la préforme et la partie destinée à supporter des contraintes élevées, notamment la partie comportant les dentures, s'il y en a.

Suivant un autre mode de réalisation, il est prévu des dentures et on prévoit dans le moule de l'instrument des moyens pour boucher les ouvertures des cavités de moulage des dentures et lorsque le moule est entièrement rempli de la matière de moulage à l'exception des cavités des dentures, les moyens de bouchage sont retirés pour laisser passer la matière de moulage dans les cavités.

Suivant ce mode de réalisation, il n'y a alors pas d'interface entre les matières plastiques de la préforme et de la partie destinée à supporter les contraintes, notamment la partie à dentures. En particulier, les moyens de bouchage peuvent comporter des moyens à ressorts qui sont repoussés par la pression de la matière de moulage qui s'accumule dans le moule pour ainsi libérer les cavités de moulage des dentures, pression accumulée qui, après libération des cavités de moulage, pousse efficacement la matière plastique de moulage dans les cavités de dentures, pour ainsi obtenir des dentures correspondant exactement à la forme des cavités.

Suivant un mode de réalisation préféré de l'invention, la préforme est obtenue par une étape de surmoulage préalable autour d'un insert métallique, notamment une tige métallique, d'un corps intermédiaire en matière plastique pour ainsi obtenir la préforme constituée de la pièce métallique entourée par de la matière plastique.

Suivant un autre mode de réalisation avantageux, la préforme est constituée entièrement en matière métallique.

La présente invention se rapporte également à un instrument chirurgical d'enlèvement de matière osseuse tel que défini aux revendications 8 à 15.

De préférence, la hauteur de la ou de chaque denture, c'est-à-dire la distance entre son sommet et ladite une surface est supérieure ou égale à 1 mm, notamment est comprise entre 1 mm et 8 mm.

De préférence, le rapport de la hauteur sur la largeur est supérieur à 0,2, notamment est comprise entre 0,3 et 2.

De préférence, la au moins une denture a en section transversale un sommet en pointe ou anguleux.

De préférence, l'angle formé par la denture au sommet est compris entre 50° et 120°, notamment entre 60° et 110°.

La présente invention se rapporte également à un dispositif ou instrument chirurgical comportant une partie intérieure et une partie extérieure destinée à supporter des contraintes élevées lors de l'enlèvement de la matière à l'aide de l'instrument ou du dispositif, cette partie extérieure étant en matière plastique et pouvant notamment comporter une ou des dentures et la partie extérieure destinée à supporter les contraintes étant surmoulée sur la partie intérieure, notamment en l'entourant, en formant entre la matière de la partie intérieure et la matière plastique de la partie extérieure un interface.

De préférence, la partie intérieure est au moins en partie en matière plastique et cette au moins une partie est séparée de la partie extérieure en matière plastique par le dit un interface.

Suivant un mode de réalisation préféré de l'invention, la matière plastique pour former la au moins une partie de la partie intérieure est identique à la matière plastique utilisée pour la partie extérieure.

Suivant un mode de réalisation préféré de l'invention, la partie intérieure est constituée d'une pièce en matière métallique, par exemple un barreau métallique, autour de laquelle est surmoulée une matière plastique, formant ainsi une préforme constituant la partie intérieure de l'instrument chirurgical, la partie extérieure en matière plastique étant surmoulée sur ou autour de la préforme.

De préférence, deux trous borgnes creusés dans la partie extérieure en matière plastique débouchent à l'extérieur de l'instrument, les fonds respectifs des trous borgnes étant formés par la matière plastique de la préforme.

Ces deux trous borgnes correspondant aux deux pions qui maintiennent la préforme dans le moule lors du moulage de la partie extérieure en matière plastique.

A titre d'exemple, on décrit maintenant un mode de réalisation préféré de l'invention en se rapportant au dessin dans lequel :
- la Figure 1: représente une tige métallique destinée à faire partie d'une râpe de hanche suivant l'invention ;
- la Figure 2: représentent la préforme obtenue après surmoulage autour de la pièce métallique de la Figure 1 d'une matière plastique ;
- la Figure 3: représente une coupe dans le plan antéropostérieur de la râpe finale après surmoulage autour de la préforme de la Figure 2 de la partie destinée à attaquer l'os ;
- la Figure 4: est une vue de côté de la râpe finale ; et
- la Figure 5: est une vue de côté du moule utilisé pour former la râpe des Figures 3 et 4.

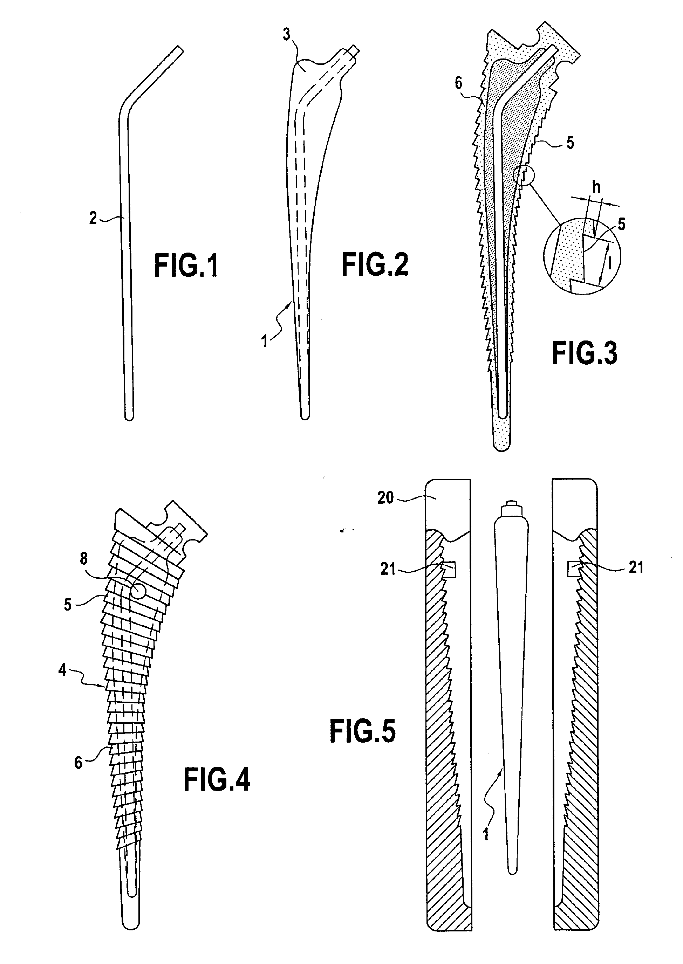

Aux Figures 1 à 4, il est représenté différentes étapes pour obtenir une râpe suivant l'invention.

A la Figure 4, il est représenté une râpe 4 suivant l'invention. Elle comporte une partie 1 intérieure ou centrale constituée d'une tige 2 en matière métallique autour de laquelle a été moulée une pièce 3 en matière plastique. La partie 3 en matière plastique et la tige 2 ainsi réunies constitue une préforme.

Cette préforme a été obtenue par moulage, notamment par injection, autour de la pièce métallique qui a été maintenue dans le premier moule par deux premiers pions latéraux. Il en résulte qu'il est formé dans cette préforme 1 deux trous débouchant à l'extérieur de la préforme et allant jusqu'à la tige 2. Ces deux trous correspondent à la forme des deux premiers pions qui maintiennent la tige 2 dans le premier moule.

Ensuite, la préforme ou partie 1 intérieure ou centrale, constituée de la tige 2 et de la partie 3 moulée autour, est elle-même positionnée dans un second moule 20 qui comporte sur ses parois internes des petites cavités destinées à former par complémentarité de forme des dents ou dentures 5 destinées à attaquer l'os lors de l'utilisation de la râpe.

Une fois la partie 1 centrale disposée dans le second moule et également maintenue par deux seconds pions 21 de part et d'autre, on coule la matière plastique sous la forme d'une couche 6 qui l'entoure complètement et qui vient remplir les cavités de formation des dents. A la sortie du moule, une fois durcie la matière plastique de moulage formant la couche 6 comportant les dents 5, on obtient la râpe 4 finale. Elle comporte sur les côtés deux trous borgnes 8 traversant la couche 6 correspondant aux seconds pions 21 de maintien. Les trous 8 débouchent à l'extérieur et leur fond est formé par la matière de la pièce 3 centrale.

Ces trous borgnes correspondant aux seconds pions de maintien de la pièce centrale dans le second moule lors du surmoulage de la couche 6 n'ont plus pour fond, comme ceux de la préforme, la pièce métallique 2 mais la partie plastique de la préforme 1. Ainsi, l'avantage est que lors du lavage de la râpe, notamment avec un liquide, notamment de l'eau, ce dernier ne peut plus venir en contact direct avec la pièce métallique 2 à l'intérieur de la râpe, empêchant l'apparition d'une corrosion toujours nuisible à la biocompatibilité de la râpe.

Suivant l'invention, on peut réaliser la pièce 3 entièrement en matière plastique, c'est-à-dire sans prévoir la pièce 2 métallique de rigidification. De même à la place de prévoir une matière plastique seule ou entourant une pièce métallique pour la pièce 3, on pourrait prévoir une unique pièce métallique.

Dans le cas où la pièce 3 est réalisée en matière plastique, cette matière plastique peut être identique à la matière plastique utilisée pour la couche 6 formant les dents 5, ou être différente en fonction des besoins.

Comme matières plastiques, on peut utiliser des polyoxyméthylènes, des polyamides, des polyéthylènes, des polymères liquicristallins, des polyvinylènes fluorures, du PMMA, mais également des polyarylamides semi-aromatiques, semi-cristallines.

De préférence, dans le cas où la matière plastique choisie n'est pas suffisamment dure pour râper de l'os, il est préférable de la traiter aux rayons β ou γ pour augmenter sa dureté et permettre ainsi son utilisation en tant que râpe pour râper de l'os. De préférence, le rayonnement γ se fait par exposition à une dose de 25 à 50 kGray. Si l'on conserve des doses appropriées, l'effet du rayonnement γ est d'une part de stériliser la pièce évitant ainsi d'avoir à la restériliser et d'autre part de durcir la matière plastique pour lui permettre de râper de l'os. En outre, une fois cette première stérilisation par rayons γ utilisée, il n'est plus possible de restériliser la râpe, notamment en la plaçant dans une autoclave, ce qui ainsi évite une réutilisation abusive de la râpe. Le chirurgien n'a plus comme solution que de la jeter après utilisation.

Suivant l'invention, les dents sont parfaitement formées. En effet, on s'est rendu compte pour la première fois que lorsqu'on souhaitait réaliser une râpe de ce genre en matière plastique, un problème se posait sur les dents qui étaient peu tranchantes. Ceci était dû, et ce sont les inventeurs qui s'en sont rendus compte, au fait que les volumes des cavités de moulage des dents étant de petite dimension, lorsqu'on coule la matière plastique dans un grand volume, les cavités ont tendance à ne pas se remplir, le volume étant trop petit, et il se forme un effet de vide dans le volume du moule. Il en résultait dans l'art antérieur que les dents n'étaient pas convenablement formées empêchant ainsi toute possibilité d'utilisation de la râpe. Suivant l'invention, au contraire, on obtient des dents très bien "aiguisées". En effet, la pression de la matière plastique formant la couche de peau 6 est beaucoup plus forte pour pousser la matière plastique dans les cavités de moulage des dents 5 puisque le volume total de la couche 6 est bien plus faible que le volume total de la râpe, au contraire de ce qui était le cas dans les systèmes de moulage de l'art antérieur.

On a décrit le principe de fabrication d'une râpe suivant l'invention. Bien évidemment, le même principe peut s'appliquer à d'autres dispositifs de coupe destinés à attaquer de l'os et comportant des dents ou ayant des parties destinées à supporter une forte contrainte liée à la coupe de l'os. Ainsi, le même principe peut s'appliquer à des fraises à cotyle ou autres dispositifs de guide de coupe. En particulier, le volume de la pièce de la couche 6 formant peau comportant les parties 5 de dents destinées à attaquer l'os est très inférieur au volume total de la râpe et également très inférieur au volume de la pièce intérieure qu'elle entoure. Ainsi, le rapport entre le volume défini par la surface extérieure de la couche 6 formant les dents 5 est au moins dix fois supérieur au volume de la couche 6 elle-même. De préférence, il est au moins cent fois supérieur. Entre la couche 6 et la partie 3 centrale, il est formé entre les matières plastiques une interface. Cette interface est présente que les deux matières soient identiques ou non.

La largeur 1 de la ou chaque denture 5, c'est-à-dire la dimension en largeur la plus petite au niveau de ladite une surface de l'instrument dont elle est issue, est inférieure à 8 mm, de préférence comprise entre 1 et 7 mm, notamment est comprise entre 2 et 5 mm, plus préférablement entre 3 et 4, par exemple 3,5 mm.

La hauteur (h) de la ou de chaque denture, c'est-à-dire la distance entre son sommet et ladite une surface, est supérieure à 0,5 mm, notamment est comprise entre 1 et 8 mm, par exemple est égales à 1 mm.

Le rapport de la largeur sur la hauteur est inférieure à 0,2, notamment compris entre à 0,15 et 2,0, par exemple égal à 1/3,5, soit 0,285.

En outre, l'épaisseur de la couche 6 de matière plastique est, hors les dents, comprise de préférence entre 0,8 mm et 5 mm. En particulier, le rapport de cette épaisseur de la couche hors les dents sur la hauteur des dents est inférieur à 10, de préférence inférieur à 5, notamment compris entre 5 et 0,5.

On peut également réaliser une fraise à cotyle suivant le même principe que la râpe des Figures 1 à 4. Elle comporte ainsi une partie centrale comportant, notamment en son sein, une tige métallique et une peau formant les dents destinées à attaquer l'os.

En outre, suivant un autre mode de réalisation, on peut prévoir dans le moule un système qui obture les ouvertures des cavités de moulage et ne les ouvre que sous l'effet de la pression de la matière plastique de moulage, lorsque seules restent non remplis les volumes des cavités de moulage des dentures. Il résulte d'un tel procédé que la matière de moulage pénètre bien dans ces cavités. On parvient ainsi à fabriquer des dents au profil mince souhaité.

Avec ce procédé, le moulage peut se faire en une seule étape et on obtient non seulement des dents parfaitement formées mais également le fait, si on le souhaite, qu'il n'y ait pas d'interface formé dans la matière plastique ou entre des matières plastiques.

On peut également, toujours dans le cadre de la présente invention, faire en sorte que la préforme, notamment métallique, ressorte de la peau en certains endroits, par exemple pour former des dents.

## Revendications

1. Procédé de fabrication par moulage d'un instrument ou d'un dispositif chirurgical pour attaquer de l'os pour en enlever une partie, dont une partie destinée à supporter des contraintes élevées liées à cette attaque est en matière plastique, cette dite une partie plastique pouvant notamment comporter au moins une denture, de préférence une pluralité de dentures faisant saillie à l'extérieur de l'instrument, et destinée(s) à attaquer l'os directement, dans lequel :
- on prépare un moule correspondant à la forme finale de l'instrument,
- on réalise préalablement dans le moule une première partie de l'instrument qui ne comporte pas la partie plastique destinée à supporter les contraintes élevées, par exemple en réalisant préalablement cette première partie sous la forme d'une préforme et en l'insérant dans le moule, de manière à laisser non rempli dans le moule sensiblement uniquement le volume correspondant à ladite partie plastique destinée à supporter les contraintes, et
- on réalise par moulage ladite partie plastique destinée à supporter les contraintes élevées, notamment sous la forme d'une couche mince, en remplissant le volume laissé entre la première partie et le moule, pour ainsi obtenir par moulage l'instrument ou dispositif chirurgical.

2. Procédé suivant la revendication 1, **caractérisé en ce que** la partie plastique comporte au moins une denture, de préférence une pluralité de dentures faisant saillie à l'extérieur de l'instrument et destinée(s) à attaquer l'os directement.

3. Procédé suivant la revendication 1 ou 2, **caractérisé en ce que** on réalise la première partie par moulage dans le moule en obturant la ou les zones ou cavités de moulage de la partie plastique destinée à supporter les contraintes, puis on ouvre cette ou ces zones et cavité pour permettre l'introduction de la matière de moulage dans ces zones ou cavités pour former ladite matière plastique destinée à supporter les contraintes.

4. Procédé suivant l'une des revendications 1 à 3, **caractérisé en ce que** la première partie est une partie intérieure et la partie plastique entoure la première partie.

5. Procédé suivant l'une des revendications 1 à 4, **caractérisé en ce que** la partie intérieure est réalisée sous la forme d'une préforme.

6. Procédé suivant la revendication 5, **caractérisé en ce que** la préforme est constituée sous la forme d'une tige métallique insérée dans le moule avant moulage de la partie plastique.

7. Procédé suivant l'une des revendications 1 à 6, **caractérisé en ce que** le volume de la première partie est plus grand que le volume de la partie plastique destinée à supporter les contraintes élevées, notamment est nettement plus grande.

8. Instrument chirurgical d'enlèvement de matière osseuse pouvant être obtenu par le procédé suivant l'une des revendications précédentes, ayant une surface de laquelle est issue au moins une denture, de préférence des dentures, en matière plastique destinées à venir en contact avec l'os pour en enlever une partie, la ou les dentures étant réalisées par moulage, notamment moulage par injection, **caractérisé en ce que** la largeur (1) de la ou de chaque denture (5), c'est-à-dire la dimension en largeur la plus petite au niveau de ladite une surface de l'instrument dont elle est issue, est comprise entre 1 mm et 7 mm, de préférence comprise entre 2 mm et 5 mm, plus préférablement comprise entre 3 mm et 4 mm.

9. Instrument chirurgical suivant la revendication 8, **caractérisé en ce que** la hauteur (h) de la ou de chaque denture, c'est-à-dire la distance entre son sommet et ladite une surface, est supérieure ou égale à 1 mm, notamment est comprise entre 1 mm et 8 mm.

10. Instrument chirurgical suivant la revendication 9, **caractérisé en ce que** le rapport de la hauteur sur la largeur est supérieur à 0,2, notamment est comprise entre 0,3 et 2,0.

11. Dispositif ou instrument chirurgical destiné à attaquer de l'os pour en enlever une partie suivant l'une des revendications 8 à 10, **caractérisé en ce qu'**il comporte une partie intérieure et une partie extérieure destinée à supporter des contraintes élevées lors de l'enlèvement de la partie d'os à l'aide de l'instrument ou du dispositif, cette partie extérieure étant en matière plastique, la partie extérieure destinée à supporter les contraintes est surmoulée sur la partie intérieure, notamment en l'entourant, en formant entre la matière de la partie intérieure et la matière plastique de la partie extérieure un interface.

12. Instrument chirurgical suivant la revendication 11, **caractérisé en ce que** la partie intérieure est au moins en partie en matière plastique et cette au moins une partie est séparée de la partie extérieure en matière plastique par le dit un interface.

13. Instrument chirurgical suivant l'une des revendications 11 ou 12, **caractérisé en ce que** la partie intérieure est constituée d'une pièce en matière métallique, par exemple un barreau métallique, autour de laquelle est surmoulée une matière plastique, formant ainsi une préforme constituant la partie intérieure de l'instrument chirurgical, la partie extérieure en matière plastique étant surmoulée sur ou autour de la préforme.

14. Instrument chirurgical suivant l'une des revendications 8 à 13, **caractérisé en ce que** deux trous borgnes creusés dans la partie extérieure en matière plastique débouchent à l'extérieur de l'instrument, les fonds respectifs des trous borgnes étant formés par la matière plastique de la préforme.

15. Instrument chirurgical suivant l'une des revendications 8 à 14, **caractérisé en ce qu'**il s'agit d'une râpe fémorale.
